Europäisches Patentamt

(19) European Patent Office

Office européen des brevets

(11) Numéro de publication: **0 132 480**
**B1**

(12) **FASCICULE DE BREVET EUROPEEN**

(45) Date de publication du fascicule du brevet:
**27.01.88**

(21) Numéro de dépôt: **83420136.0**

(22) Date de dépôt: **29.07.83**

(51) Int. Cl.⁴: **A 61 J 3/00,** A 23 G 3/26

(54) Dispositif pour fabriquer en vrac des produits en sucre ou analogue, par exemple pour la pharmacie homéopathique et produits ainsi obtenus.

(43) Date de publication de la demande:
**13.02.85 Bulletin 85/7**

(45) Mention de la délivrance du brevet:
**27.01.88 Bulletin 88/4**

(84) Etats contractants désignés:
**AT BE CH DE FR GB IT LI LU NL SE**

(56) Documents cités:
**DE - B - 2 423 933**
**DE - B - 2 731 351**
**FR - A - 1 411 335**
**FR - A - 2 137 170**
**FR - A - 2 374 968**

(73) Titulaire: **BOIRON, 20 rue de la Libération,**
**F-69110 Sainte Foy Les Lyon (FR)**

(72) Inventeur: **Abecassis, Jacky, 8 rue du Prieuré,**
**F-69130 Ecully (FR)**
Inventeur: **Baume, Bernard, Parc de Vieux Logis Route**
**du Mont Verdun, F-69760 Limonest (FR)**
Inventeur: **Favier, André Marcel, Rue des Gabettes**
**Dagneux, F-01120 Montluel (FR)**

(74) Mandataire: **Laurent, Michel, Cabinet Michel**
**Laurent 20 rue Louis Chirpaz B.P. 32, F-69131 Ecully**
**Cédex (FR)**

## Description

La présente invention est relative à un dispositif d'un type nouveau destiné à permettre la fabrication en vrac d'une grande quantité de petites billes, à partir d'un matériau susceptible d'être pulvérisé à l'état liquide, fondu ou en solution dans un solvant. On sait que des billes de ce type, fabriquées en sucre, sont utilisées pour servir de base aux médicaments homéopathiques: lorsque leur diamètre est de l'ordre de 5 à 6 millimètres, ils constituent des «granules», alors qu'on les désigne par la dénomination «globules», lorsque leur diamètre est de l'ordre de 1,5 millimètre.

A ce jour, il est connu de fabriquer les billes en sucre des granules ou globules homéopathiques en utilisant un mélangeur à cuve rotative, connu sous le nom de «turbine DUMOULIN». Cet appareil connu comporte une cuve rotative, à l'intérieur de laquelle on fait se succéder des cycles opératoires dont chacun comprend:

– une pulvérisation d'un sirop de sucre sur la masse en mouvement des billes en cours de formation:
– un temps d'enrobage, pendant lequel la turbine continue à tourner;
– un temps de distribution de poudre, pendant lequel on introduit dans la cuve en rotation, du sucre de lait en poudre, qui vient s'agglomérer sur les billes dont il augmente ainsi le diamètre;
– un temps de séchage à air chaud dont la durée dépend de l'état hygrométrique de l'atmosphère environnante.

En pratique, on constate que, pour un cycle dont la durée est de six à douze minutes, le temps de séchage à air chaud occupe, à lui seul, de cinq à onze minutes, suivant l'hygrométrie ambiante. Autrement dit, lorsqu'on procède à un séchage à l'air chaud, le temps de séchage occupe, à lui seul, de 83 à 91% du temps de fabrication. Si l'on sait que le temps total pour la fabrication des granules est supérieur à 20 jours, on conçoit l'importance de la durée de séchage dans le prix de revient.

Par ailleurs, on constate que, plus le cycle total de fabrication est long, plus le produit se détériore. En effet, le sucre est abrasif, si bien que les produits secs les premiers usent les autres, ce qui fait apparaître sur certaines billes de sucre, des phénomènes de transparence, accompagnés d'un dégagement de poudre. En pratique, on constate que la clientèle a tendance à assimiler les produits transparents, à des granules qui auraient été stockés trop longtemps, bien que cela soit souvent inexact. Quoi qu'il en soit, l'aspect transparent est à éviter sur le produit final, dont une proportion non négligeable doit être rebutée, lorsqu'on utilise les techniques de fabrication actuellement connues.

Dans le brevet FR-A-2 374 968, on a proposé un procédé d'enrobage du type en question, dans lequel on évapore l'eau de la solution déposée au moyen de radiations électromagnétiques émises par un magnétron disposé à l'extérieur de la cuve, dans laquelle les radiations sont amenées au moyen d'un guide d'ondes fixe. Cette disposition entraîne malheureusement une déperdition appréciable et n'assure pas une répartition et une diffusion homogènes des ondes dans la cuve, notamment avec des cuves de gros tonnages.

La présente invention a pour but d'éviter ces inconvénients, en réalisant un dispositif permettant de fabriquer en vrac des billes de sucre ou analogues, dont le temps total de fabrication soit seulement environ 40% du temps actuellement nécessaire.

Le dispositif selon l'invention peut être utilisé pour fabriquer toutes sortes de produits en sucre ou analogues, c'est-à-dire non seulement des globules ou granules pour les médicaments homéopathiques, mais également des bonbons, des dragées, ou d'autres articles de confiserie, les produits ainsi fabriqués n'étant donc pas forcément de forme rigoureusement sphérique, mais simplement arrondie et convexe.

Le dispositif selon l'invention pour fabriquer les produits en sucre sous forme de globules, granules, billes ou analogues, comprend:

– une cuve rotative, à l'intérieur de laquelle se trouve au moins un pulvérisateur de produit liquide et qui est rotative autour d'un axe théorique, cette cuve présentant:

. une première canalisation pour amener de l'air sec à l'intérieur de la cuve rotative,
. une deuxième canalisation pour extraire l'air chargé d'humidité;
– au moins un générateur à micro-ondes du genre magnétron, qui émet des ondes électromagnétiques dirigées vers la masse des billes en cours de formation à l'intérieur de la cuve rotative.

Il se caractérise:

– d'une part, en ce que les magnétrons sont juxtaposés le long d'un rampe orientée le long de l'axe théorique de rotation de la cuve;
– et d'autre part, en ce que cette rampe est solidaire d'un chariot susceptible d'avancer ou de reculer le long d'un chemin de roulement horizontal, ce qui permet d'introduire cette rampe à l'intérieur de la cuve ou au contraire de l'en extraire.

Dans une forme de réalisation avantageuse, l'ouverture axiale de la cuve coopère avec un couvercle amovible dont la fenêtre centrale est constituée par un verrre conducteur placé à l'extérieur d'une grille métallique, tandis qu'autour de cette fenêtre sont répartis des pièges concentriques, dont la longueur axiale correspond au quart de la longueur d'ondes des micro-ondes émises par les magnétrons.

Le dessin annexé donné à titre d'exemple non limitatif permettra de mieux comprendre les caractéristiques de l'invention.

La figure 1 est une vue extérieure schématique montrant une forme de réalisation de l'invention pour équiper une cuve rotative cylindrique de grande capacité.

La figure 2 est une vue avec arrachement partiel.

La figure 3 est une coupe suivant VI–VI (figure 1) illustrant la structure du couvercle amovible.

La figure 4 est une vue latérale de l'ensemble de l'installation, illustrant notamment les déplacements possibles de la rampe à magnétrons.

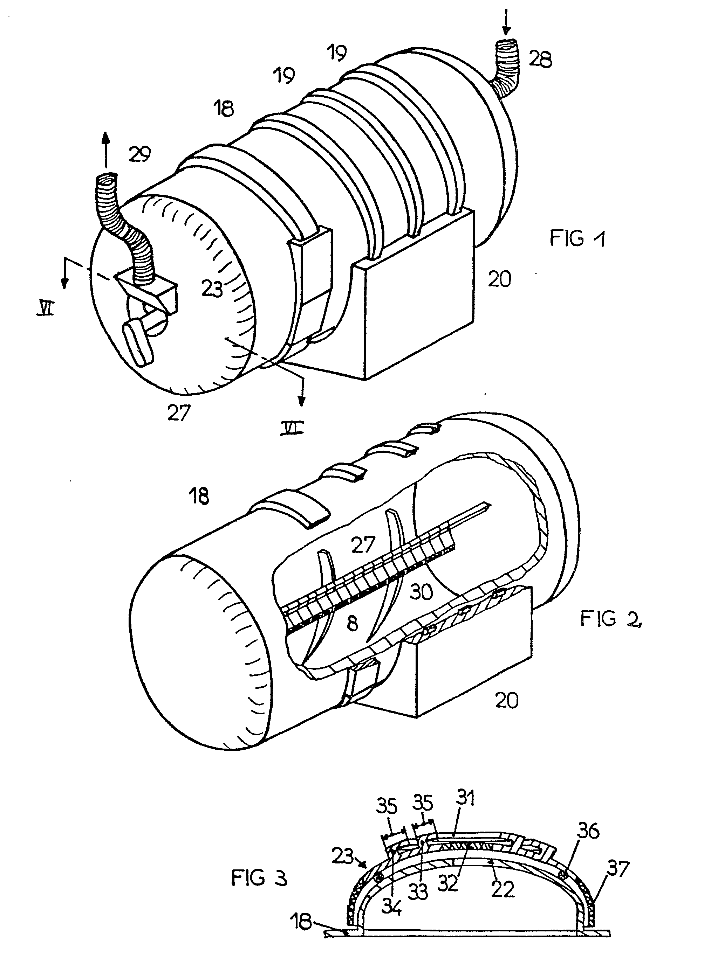

On a représenté sur les figures 1 à 4, une réalisation prévue pour fabriquer à la fois une grande quantité de billes en vrac (par exemple 750 kilos).

La cuve rotative 18 a la forme d'un cylindre et elle possède des ceintures 19 constituant des chemins de roulement qui tournent sur les galets non représentés d'un socle fixe 20. Ainsi, la cuve 18 tourne autour d'un axe fictif horizontal 21.

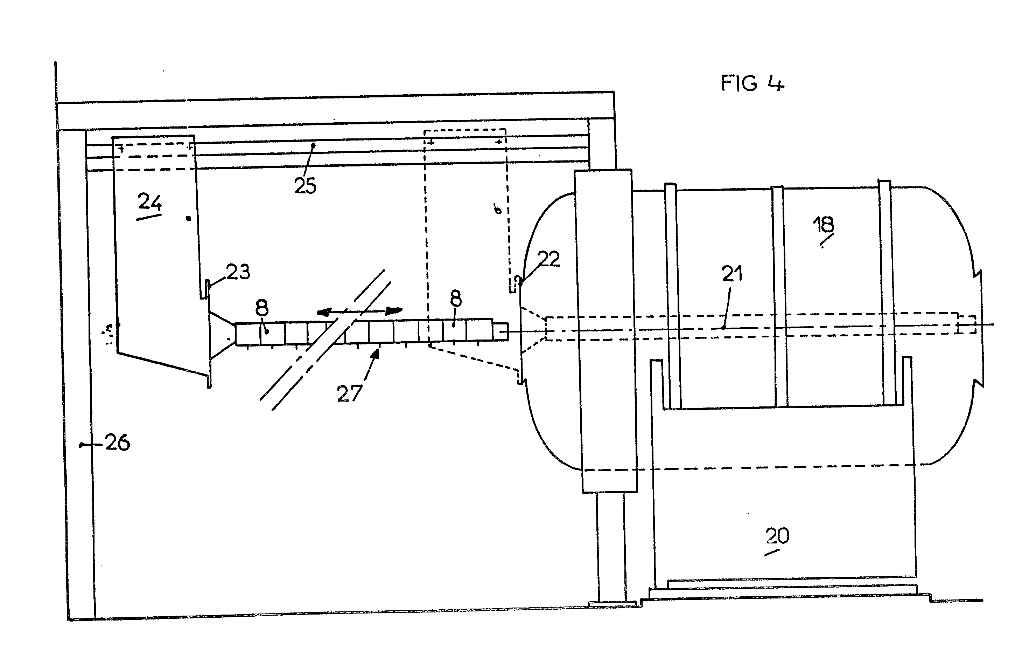

A l'une de ses extrémités, la cuve 18 comporte une ouverture centrale 22 que peut venir obturer un couvercle amovible 23. Ce dernier est solidaire d'un chariot 24 dont la partie supérieure est susceptible de se déplacer le long du chemin de roulement horizontal 25 d'un portique fixe 26. Dans sa partie centrale, le couvercle 23 est solidaire d'une rampe à micro-ondes 27, dirigée suivant l'axe horizontal 21. Cette rampe 27 est constituée par une juxtaposition de magnétrons (8). Chaque magnétron (8) comprend un générateur dont l'antenne émet des ondes électromagnétiques, par exemple sur une fréquence de 2450 mégaherz, sur une longueur d'ondes de douze centimètres. Lorsqu'on fait reculer le chariot (24) sur son chemin de roulement (25) (position illustrée à gauche en traits pleins sur la figure 4), la rampe à magnétrons (27) se trouve entièrement située hors de la cuve (18). On peut accéder à l'intérieur de celle-ci.

Au contraire, pendant le fonctionnement, le chariot (24) occupe la position de fermeture illustrée en traits interrompus sur la figure 4, le couvercle (23) venant alors obturer l'ouverture (22).

L'ensemble est complété par des canalisations d'arrivée d'air sec (28) et d'extraction d'air humide (29).

L'ensemble est complété par des organes de type connu, tels que des aubages de malaxage (30) solidaires de la paroi interne de la cuve rotative 18, afin de faire circuler les billes de sucre pendant la rotation de la cuve. De même, des pulvérisateurs non représentés sont prévus pour pulvériser du sirop de sucre ou de la poudre de sucre de lait.

Le couvercle 23 est préférablement réalisé comme indiqué sur la figure 3. A cet effet, il comporte une fenêtre centrale 31 équipée d'un verre conducteur, par exemple revêtu sur une face d'une couche métallique fine et transparente, ou bien d'une matière plastique chargée en carbone. A l'intérieur de cette fenêtre 31 se trouve une grille métallique 32, destinée à arrêter les micro-ondes.

Par ailleurs, on dispose autour de la fenêtre 31, des reliefs annulaires constituant chacun un relief quart d'onde, tels que 33 et 34. Dans l'exemple illustré sur les dessins, la longueur radiale 35 de chacun de ces pièges annulaires sera par exemple égale à 3 centimètres.

Autour d'eux, on prévoit un absorbant, constitué par exemple par un jonc en caoutchouc au silicone 36. Enfin, la périphérie du couvercle 23 se termine par une bavette 37 en caoutchouc au silicone. Grâce à cette disposition on permet à l'opérateur d'observer l'intérieur de la cuve 18 à travers la fenêtre 31, sans être incommodé par les micro-ondes réfléchies.

## Revendications

1. Dispositif pour fabriquer des produits en sucre (13), sous forme de globules, granules, billes ou analogues, comprenant:
– une cuve rotative (18), à l'intérieur de laquelle se trouve au moins un pulvérisateur de produit liquide, et qui est rotative autour d'un axe théorique (21), cette cuve présentant:
· une première canalisation (28) pour amener de l'air sec à l'intérieur de la cuve rotative (18);
· une deuxième canalisation (29) pour extraire l'air chargé d'humidité,
– au moins un générateur à micro-ondes (8) du genre magnétron qui émet des ondes électromagnétiques dirigées vers la masse des billes (13) en cours de formation à l'intérieur de la cuve rotative (18),
caractérisé:

– en ce que les magnétrons (8) sont juxtaposés le long d'une rampe (27) orientée le long de l'axe théorique (21) de rotation de la cuve (18);
– et en ce que cette rampe (27) est solidaire d'un chariot (24) susceptible d'avancer ou de reculer le long d'un chemin de roulement (25) horizontal, ce qui permet d'introduire cette rampe (27) à l'intérieur de la cuve (18) ou au contraire de l'en extraire.

2. Dispositif selon la revendication 1, caractérisé en ce que l'ouverture axiale (22) de la cuve (18) coopère avec un couvercle amovible (23) dont la fenêtre centrale (31) est constituée par un verre conducteur placé à l'extérieur de la grille métallique, tandis qu'autour de cette fenêtre (2), sont répartis des pièges concentriques dont la longueur radiale correspond au quart de la longueur d'ondes des micro-ondes émises par les magnétrons (8).

## Patentansprüche

1. Vorrichtung zum Herstellen von Zuckerprodukten (13) in Form von Kügelchen, Granalien, Blöcken oder dgl., mit einem drehbaren Kessel (18), in dessen Innerem sich zumindest ein Pulverisierungsapparat für flüssige Produkte befindet und der um eine gedachte Achse (21) drehbar ist, wobei der Kessel eine erste Leitung (28) zum Zuführen von trockener Luft in das Innere des drehbaren Kessels; eine zweite Leitung (29) zum Abführen von mit Flüssigkeit geladener Luft, und zumindest einen Magnetron-Mikrowellengenerator (8) aufweist, der elektromagnetische Wellen ausstrahlt, die auf die Masse an Blöcken (13) während deren Bildung im Inneren des drehbaren Kessels (18) gerichtet sind, dadurch gekennzeichnet, dass die Magnetrone (8) längs einer Rampe (27) nebeneinandergelegt sind, die längs der gedachten Achse (21) der Drehung des Kessels (18) ausgerichtet ist; und dass die Rampe (27) mit einem Schlitten (24) fest verbunden ist, der in der Lage ist, längs eines horizontalen Rollpfades (25) hin- und herzufahren, wodurch es ermöglicht ist, die Rampe (27) in das Innere des Kessels (18) einzuführen oder, im Gegensatz dazu, sie herauszuziehen.

2. Vorrichtung nach Anspruch 1, dadurch gekennzeichnet, dass die axiale Öffnung (22) des Kessels (18) mit einem abnehmbaren Deckel (23) zusammenwirkt, dessen mittiges Fenster (31) durch ein leitfähiges Glas gebildet ist, das an der Aussenseite des metallischen Gitters angeordnet ist, wobei um das Fenster (2) konzentrische Glieder verteilt sind, deren radiale Länge einem Viertel der Wellenlänge der durch die Magnetrone (8) ausgesandten Mikrowellen entspricht.

**Claims**

1. Device for the production of sugar products (13), such as globules, granules, balls or the like, comprising:

- a rotary drum (18) within which is located at least one atomizer for a liquid product, and which is rotating around a theoretical axis (21), this drum comprising:
  . a first duct (28) for blowing dry air into said rotary drum (18);
  . a second duct (29) for removing moist air,
- at least one microwave generator (8) of the magnetron type which emits electromagnetic waves directed towards the mass of balls (13), which are being formed within the rotary drum (18);
  characterized:

- by the fact that the magnetrons (8) are juxtaposed along a ramp (27) oriented along the theoretical rotary axis (21) of the drum (18);
- by the fact that this ramp (27) is integral with a carriage (24) which can advance or move back along a horizontal rolling track (25), permitting the ramp (27) to be introduced into the interior of the drum (18) or, on the contrary, to be withdrawn from it.

2. Device according to claim 1, characterized by the fact that the axial opening (22) of the drum (18) cooperates with a removable cover (23), the central window (31) of which is constituted by a conductive glass placed externally of a metallic grid, while around this window (2) are distributed concentric traps, the radial length of which corresponds to a quarter of the wavelength of the microwaves emitted by the magnetrons (8).

FIG 1

FIG 2

FIG 3

FIG 4